# EUROPEAN PATENT SPECIFICATION

(11) **EP 3 463 332 B1**
(45) Date of publication and mention of the grant of the patent: **21.04.2021**
(21) Application number: 17711109.3
(22) Date of filing: 10.03.2017
(51) Int. Cl.: A61K 31/202, A61P 27/14, A61P 37/08

(54) **DGLA IN THE PROPHYLAXIS OF ALLERGIC DISEASE**
DGLA IN DER PROPHYLAXE VON ALLERGIEERKRANKUNGEN
DGLA DANS LA PROPHYLAXIE DE MALADIES ALLERGIQUES

(30) Priority: 01.06.2016 EP 16172431
(43) Date of publication of application: 10.04.2019
(73) Proprietor: Société des Produits Nestlé S.A., 1800 Vevey (CH)
(72) Inventor: ECKHARDT, Erik, 03100 Montlucon (FR); NEMBRINI, Chiara, CH-1081 Montpreveyres (CH); JOURDAIN, Laureline, Glendale, CA 91208 (US)
(74) Representative: Krishnan, Sri
(86) International application number: PCT/EP2017/055680
(87) International publication number: WO 2017/207124

(56) References cited:
- US-A- 5 591 446
- US-A- 6 150 411

## Description

### FIELD OF INVENTION

The present invention relates to a composition for use in the prophylaxis of allergy and allergic diseases. In particular the present invention relates to the prophylaxis of allergy and allergic diseases in the offspring of a subject through administration of a composition comprising dihomo-gamma-linolenic acid, to said subject pre-pregnancy, during pregnancy and/or during lactation.

### BACKGROUND

Allergies and allergic diseases may be best described as hypersensitivity of the body and in particular the immune system, against normally benign foreign materials. Over the last few decades there has been an increasing number of children and adults suffering from allergies and allergic diseases e.g. eczema, atopic dermatitis, allergic rhinitis and asthma; this ever increasing numbers of patients poses a huge burden on healthcare systems worldwide.

Despite extensive research treatment options for sufferers of allergies or allergic diseases are limited. Widely-used therapies often only provide symptomatic relief (e.g. antihistamines, decongestants, and steroids) and said treatments may suffer from draw backs e.g. secondary effects such as drowsiness. Known preventative treatments e.g. immunotherapy or desensitisation therapy, may be expensive and are only of varying success or not applicable e.g. for those allergies caused by food.

US 5 591 446 discloses atopy-prophylaxis dietary supplement comprising at least one substance selected from the group consisting of .gamma.-linolenic acid; and dihomo-.gamma.-linolenic acid. This reference further discloses two compositions under table 2 and 3 (for pregnant or nursing mothers a composition comprising GLA or DGLA or GLA+DGLA), but is silent about omega-3 polyunsaturated fatty acids.

US 6 150 411 relates to combating dyslexia or inadequate night vision or dark adaptation in dyslexics or normal individuals, by administering DHA or a precursor n-3 EFA.

Accordingly, there exists a need to provide a therapy, especially a prophylactic therapy for allergic diseases or conditions. In particular, it would be desirable to provide a therapy that can prevent or reduce the risk of development of allergies.

The inventors have now surprisingly found that dihomo-gamma-linolenic acid (DGLA), or a composition comprising DGLA, may prevent or reduce the severity of allergic disease in an offspring of a subject when administered to said subject during pregnancy and/or lactation.

### SUMMARY OF THE INVENTION

The invention is set out in the claims. Any references in the description to methods of treatment refer to the compositions of the present invention for use in a method of prophylactic treatment of the human (or animal) body by therapy. The invention provides a composition comprising DGLA for use in the prophylaxis of allergic disease in an offspring of a mammalian subject, comprising administration of the composition to said subject pre-pregnancy and/or during pregnancy and/or during lactation and preferably wherein said composition is a composition enriched in DGLA; wherein said composition also contains an omega-3 polyunsaturated fatty acid, selected from the group consisting of DHA and EPA or a combination of DHA and EPA; wherein said DGLA is comprised in said composition in a concentration of at least 3wt% relative to the total fatty acid content of the composition and more preferably in a concentration of at least 5wt%, at least 10wt%, at least 20wt%, at least 30wt%, at least 35wt%, or at least 40wt% relative to the total fatty acid content of the composition; and wherein the concentration of DGLA is greater than the concentration of DHA or EPA.

In one embodiment the composition further comprises omega-6 polyunsaturated fatty acid selected from the group consisting of LA and GLA or a combination of LA and GLA.

In one embodiment the composition comprises DGLA, GLA and LA and wherein the concentration of DGLA is greater than GLA and the concentration of GLA is greater than the concentration of LA.

The composition of the present invention is a maternal nutritional composition and preferably selected from the group consisting of: pre-pregnancy supplement, pregnancy supplement or lactation supplement.

The prophylactic effect of composition comprising DGLA may persist after administration has ceased and said effect in the offspring may be long term and may extend through infancy, childhood and/or into adulthood e.g. 3months, 6months, 12months, 1 year, 5 years, 10 years, 20 years or more after administration to the subject has ceased.

The composition comprising DGLA may be for maternal administration and in the case of the composition may be a maternal nutritional composition and in particular may be a pre-pregnancy supplement, a pregnancy supplement or a lactation supplement.

The composition comprising DGLA may be particularly effective for use in the prophylaxis of allergy and allergic diseases selected from the group consisting of: an atopic disorder including hereditary atopic disorder and Type 1 allergic disease including IgE mediated allergic disease (e.g. due to eosinophil infiltration and/or mast cell sensitization or activation) and asthma, allergic arthritis, allergic asthma, allergic bronchitis, allergic conjunctivitis, allergic keratitis, allergic rhinitis, allergic sinusitis, alimentary allergy, allergic respiratory disease, animal dander allergy, atopic dermatitis, atopic eczema, atopy, bronchial asthma, contact dermatitis, dermatitis, drug allergy, eczema, food allergy (particularly selected from the group consisting of egg allergy, fish allergy, milk allergy, nut allergy, shellfish allergy, soya allergy, and wheat allergy), food hypersensitivity, hayfever, house dust mite allergy, hypersensitivity pneumonitis, hypertrophic rhinitis, insect allergy, latex allergy, mould allergy, pruritus, seasonal allergic rhinitis, and vasomotor rhinitis.

The composition comprising DGLA may be used in a method for the prophylaxis of allergic disease in an offspring of a subject comprising administering a said composition to a subject pre-pregnancy, during pregnancy and/or during lactation. The composition comprising DGLA may be administered in a therapeutically active amount.

The composition comprising DGLA may also be used in the manufacture of a composition for use in the prophylaxis of allergic disease in an offspring of a subject wherein said composition is administered to said subject pre-pregnancy and/or during pregnancy and/or during lactation. The composition comprising DGLA may be administered in a therapeutically active amount.

### DESCRIPTION OF THE DRAWINGS

Figure 1: Total serum IgE content in three experimental groups (control, fish oil, and fish oil plus DGLA oil).
Figure 2: Aspergillus-specific serum IgG1 content in three experimental groups (control, fish oil (reference), and fish oil plus DGLA oil).
Figure 3: Atopic dermatitis symptoms score in three experimental groups (control, fish oil (reference), and fish oil plus DGLA oil).
Figure 4: Jejunum mast cell numbers in three experimental groups (control, fish oil (reference), and fish oil plus DGLA oil).
Figure 5: Provoked IL4 production by cultured basophils pre-treated with fatty acids in ratios as found in fish oil (reference), DGLA oil (reference), or fish oil plus DGLA oil.
Figure 6: IL10 secretion in brachial lymph nodes

### DETAILED DESCRIPTION OF THE INVENTION

Unless otherwise indicated, references to % relate to weight %.

As used herein, unless indicated otherwise, references to weight % of a particular polyunsaturated fatty acid, e.g. DGLA, EPA, DHA, LA, GLA, etc. in a composition is a weight % relative to the total fatty acid content of the composition.

As used herein, references to specific polyunsaturated fatty acids, including DGLA, LA, GLA, EPA, DHA, etc. include esters of e.g. glycerides, including triglycerides, diglycerides and monoglycerides), alkyl esters (including methyl and ethyl esters), phospholipids and glycolipids, preferably glycerides.

### DGLA and DGLA compositions

The term subject as used herein refers to a mammalian subject such as cat, dog or human. In particular the term refers to a pregnant mammal, a lactating mammal or a mammal trying to conceive/become pregnant. More particularly the term refers to a pregnant human, a lactating human or a human trying to conceive/become pregnant.

As used herein, unless otherwise indicated, the term offspring encompasses the offspring of the subject at any stage of development including fetus, neonate, infant, child and adult stages. Preferably, in any embodiment of the present invention, the term offspring refers to the neonate, infant, child and adult stages, and more preferably the infant, child and adult stages. Preferably, in humans, the neonate stage refers to the first 28 days after birth. Preferably, in humans, the infant stage refers to period from 1 month to 24 months. Preferably, in humans, the child stage refers to the period from 2 years to 16 years. Preferably, in humans, the adult stage refers to period beyond 16 years.

The DGLA may be comprised in a composition as an active agent.

In another aspect there is provided a composition comprising DGLA. Said composition may be for use in the prophylaxis of allergic disease in the offspring of a subject wherein said composition is administered to said subject pre-pregnancy and/or during pregnancy and/or during lactation.

As used herein, unless otherwise indicated, the term DGLA includes DGLA as a free fatty acid, or fatty acid esters, including monoglycerides, triglycerides, diglycerides, phospholipids, cholesterol esters. In particular, in any aspect or embodiment of the present invention, the DGLA is in the form of its triglyceride. The DGLA employed in the invention or comprised in the composition may stem from any source for example from fish products, meat products, eggs, and microorganisms. For example, EP0399494A discloses a process for the production of a DGLA-containing oil by the fermentation of a microorganism such as a fungus selected form the group consisting of fungi belong to the genus Conidiobolus or Mortierella on a culture medium containing a compound which is an inhibitor of Δ5 desaturase inhibitor such as curcumin, anisole, methoxyphenol, dimethyoxybenzene, and eugenol.

As another example, EP0535940A discloses a process for the production of a composition containing DGLA by culturing a microorganism (e.g. fungi such as Mortierella, Pythium or Entomorphyhora, preferably Mortierella, e.g. Mortierella alpina), having the ability to produce arachidonic acid (ARA) and having reduced or lost Δ5 desaturase activity (e.g. by the addition of a Δ5 desaturase inhibitor). The composition extracted from the fermentation broth may contain a high content of DGLA. A particularly suitable composition for use according to the present invention can be prepared from Mortierella alpina following the processes disclosed in EP0535940A and Kawashima, H., et al, J. Amer. Oil Chem. Soc. (2000), 77(11), 1135-1139. Such compositions comprise a triglyceride in which about 40% of the constituent fatty acids are DGLA. Compositions prepared by the processes disclosed in e.g. EP0535940A may be particularly suitable for the DGLA composition according to the present invention.

The composition of the invention may comprise DGLA in any concentration. However, the composition of the invention may be more effective if it comprises DGLA in a concentration of at least 2wt%, at least 3wt%, at least 5wt%, at least 10wt%, at least 20wt%, at least 25wt%, at least 30wt%, at least 35wt%, or at least 40wt%, at least 50wt%, at least 60wt%, at least 70wt% at least 80wt%, at least 95wt% relative to the total fatty acid content of the composition. In particular the composition may comprise DGLA in a concentration of at least 30wt%, more particularly at least 35wt% relative to the total fatty acid content of the composition.

In particular the composition of the invention will be a composition enriched in DGLA.

The term enriched as used herein refers to a composition to which DGLA has been added and thereby to a composition wherein the concentration of DGLA is greater than that normally or naturally occurring in said composition.

In particular the composition may be a fish oil composition enriched in DGLA.

The composition comprising DGLA may take any form suitable for ingestion by the subject e.g. it may be a powdered nutritional composition, a food product, a functional food product, a drink (beverage), a dairy product, a pharmaceutical formulation, a pet food product, a nutraceutical, a nutritional supplement e.g. pre-pregnancy, pregnancy and/or lactation supplement, a food product (e.g. a powder, liquid or oil for addition to food or a food/nutritional supplement), or may be included in a food - i.e. the food product can be a food to which DGLA) or the composition comprising DGLA as described herein has been added. The composition may also be included in a pharmaceutical product (e.g. a tablet, capsule or liquid). In Particular the composition will be a pre-pregnancy, pregnancy and/or lactation supplement.

The composition may comprise one or more physiologically or pharmaceutically acceptable additives or excipients, or an ingredient commonly comprised in a particular type/form of composition e.g. pre-pregnancy, pregnancy and/or lactation supplement. Non limiting examples include: preservatives e.g. antioxidants (e.g. tocopherol, ascorbic acid) or flavourings, lipids, carbohydrates, protein, micronutrients, pharmaceutically active agents, conventional food additives such as anti-oxidants, stabilizers, emulsifiers, acidulants, thickeners, buffers or agents for pH adjustment, chelating agents, colorants, excipients, osmotic agents, pharmaceutically acceptable carriers, preservatives, sugars, sweeteners, texturizers, emulsifiers, water, and vitamins and minerals, for example, vitamins and minerals recommended by a governmental body, such as USRDA, for supplementation in pregnancy e.g. calcium, magnesium, phosphorus, iron, zinc, copper, iodine, selenium, vitamin A or retinol activity equivalent (RAE) e.g. beta carotene or a mix of carotenoids, vitamin C, vitamin B1, niacin, folic acid, biotin, vitamin E.

The composition comprising DGLA may be administered in a therapeutically active amount.

A therapeutically effective dose may be any dose that has a prophylactic effect with respect to allergic disease in the offspring of a subject to whom the DGLA comprising composition has been administered pre-pregnancy and/or during pregnancy and/or during lactation.

It is well within the purview of the skilled person to determine a therapeutically effective dose. Said dose may depend on age, size and health status of the subject, on the subject's lifestyle, as well as on its genetic heritage and whether there is a history of allergy.

An effective dose may, for example, be determined by measuring the effect of a dose on a subject's offspring's risk of developing an allergenic disorder. An effective dose should preferably result in a statistically significant decrease in said risk in comparison to the risk calculated for that of an offspring of a subject to whom no composition comprising DGLA has been administered. Allergenic marker scores such as antibody scores e.g. IgE and IgG scores, and/or skin allergy symptom scores may be used to determine and compare risks.

Particularly beneficial concentrations for humans may be those equating to a dose of DGLA of about 5-1000 mg, about 5-800 mg, about 5-500 mg, about 5-250 mg, about 5-150 mg about 5-100 mg per subject per day, in particular a dose of DGLA of about 10-1000 mg, about 10-800 mg, about 10-500 mg, about 10-250 mg, about 10-150 mg about 10-100 mg per subject per day.

More particularly concentrations equating to a dose of DGLA of about 25-1000 mg, about 25-800 mg, about 25-500 mg, about 25-250 mg, about 25-150 mg about 25-100 mg per adult per day. The composition may alternatively provide a dose of DGLA of about 50-1000 mg, about 50-800 mg, about 50-500 mg, about 50-250 mg, about 50-150 mg about 50-100 mg per subject per day.

The composition comprising DGLA may be simultaneously, sequentially or separately administered with other omega-6 PUFAs in addition to DGLA. In particular, the composition comprising DGLA, may further comprise at least one other omega-6-PUFA, the DGLA is thereby administered simultaneously to the other omega-6 PUFA.

Particular omega-6 PUFAs include linoleic acid (LA) (18:2 n-6) and gamma-linolenic acid (GLA). It may be particularly beneficial if the composition further comprises LA and/or GLA.

If the composition comprising DGLA also comprise LA and/or GLA it may be particularly beneficial if the concentration of DGLA in said composition is greater than the concentration of GLA or LA, or GLA and LA.

The composition comprising DGLA, further comprises at least one omega-3-PUFA selected from the group consisting of DHA and EPA or a combination of DHA and EPA; wherein said DGLA is comprised in said composition in a concentration of at least 3wt% relative to the total fatty acid content of the composition and more preferably in a concentration of at least 5wt%, at least 10wt%, at least 20wt%, at least 30wt%, at least 35wt%, or at least 40wt% relative to the total fatty acid content of the composition; and wherein the concentration of DGLA is greater than the concentration of DHA or EPA, the DGLA is thereby administered simultaneously to this omega-3 PUFA.

The DHA and EPA may be present in a ratio of 1:5 to 5:1.

In another aspect of the invention there is provided a method for the prophylaxis of allergic disease in an offspring comprising administering a therapeutically active amount of a composition comprising DGLA as disclosed herein, to a subject pre-pregnancy, during pregnancy and/or during lactation, and preferably during pregnancy and/or during lactation.

In another aspect of the invention there is provided a composition comprising DGLA as disclosed herein, for use in the manufacture of a composition for use in the prophylaxis of allergic disease in an offspring of a subject wherein said composition is administered to said subject pre-pregnancy and/or during pregnancy and/or during lactation.

### Allergy and Allergic Conditions

As used herein, unless otherwise indicated, prophylaxis refers to preventing a disorder, as well as reducing the risk of development or preventing the onset of a disorder. For example, prophylaxis of allergic disease includes preventing an allergic disease in an offspring, as well as reducing the risk of development of an allergic disease in an offspring.

The prophylactic effect of the invention may persist after administration of the composition has ceased and said effect in the offspring may be long term and may extend through infancy, childhood and/or into adulthood e.g. 3months, 6months, 12months, 1 year, 5 years, 10 years, 20 years or more after administration to the subject has ceased.

The invention is particularly suitable for the prophylaxis of allergic conditions in an offspring due to atopy (e.g. offspring at high risk of developing atopic conditions due to maternal and/or paternal atopy); Type 1 allergic diseases including those mediated by IgE (e.g. by eosinophil infiltration and/or mast cell sensitisation or activation and/or basophil sensitization or activation).

Thus, the compositions of the invention are especially suitable for the prophylaxis of an allergic condition in an offspring, wherein the allergic condition may be selected from the group consisting of: asthma, allergic arthritis, allergic asthma, allergic bronchitis, allergic conjunctivitis, allergic keratitis, allergic rhinitis, allergic sinusitis, alimentary allergy, allergic respiratory disease, animal dander allergy, atopic dermatitis, atopic eczema, atopy, bronchial asthma, contact dermatitis, dermatitis, drug allergy, eczema, food allergy (particularly selected from the group consisting of egg allergy, fish allergy, milk allergy, nut allergy, shellfish allergy, soya allergy, and wheat allergy), food hypersensitivity, eosinophilic esophagitis, hayfever, house dust mite allergy, hypersensitivity pneumonitis, hypertrophic rhinitis, insect allergy, latex allergy, mould allergy, pruritus, seasonal allergic rhinitis, and vasomotor rhinitis.

In any embodiment of the present invention, the composition may be particularly useful for the prophylaxis of an allergic disease selected from the group consisting of: asthma, allergic asthma, allergic bronchitis, allergic conjunctivitis, allergic keratitis, allergic rhinitis, allergic respiratory disease, animal dander allergy, atopic dermatitis, atopic eczema, atopy, contact dermatitis, dermatitis, eczema, food allergy (particularly selected from the group consisting of egg allergy, fish allergy, milk allergy, nut allergy, shellfish allergy, soya allergy, and wheat allergy), hayfever, house dust mite allergy, latex allergy, mould allergy, pruritus, and/or seasonal allergic rhinitis.

Of these, the allergic disease may especially be selected from the group consisting of: asthma, allergic asthma, allergic conjunctivitis, allergic rhinitis, atopic dermatitis, atopic eczema, dermatitis, eczema, food allergy (particularly selected from the group consisting of egg allergy, fish allergy, milk allergy, nut allergy, shellfish allergy, soya allergy, and wheat allergy), hayfever, house dust mite allergy, pruritus, and/or seasonal allergic rhinitis..

More especially, allergic disease or atopic disorder is selected from the group consisting of: asthma, allergic asthma, allergic conjunctivitis, allergic rhinitis, atopic dermatitis, atopic eczema, dermatitis, eczema, hayfever and/or seasonal allergic rhinitis. Even more particularly, the allergic disease may be selected from the group consisting of: atopic dermatitis and atopic eczema, eosinophilic esophagitis, and IgE mediated asthma.

### Administration

The composition comprising DGLA according to any aspect or embodiment of the invention, may be administered to the subject pre-pregnancy i.e. prior to conception and/or during pregnancy and/or during lactation. In particular the composition comprising DGLA may be administered to the subject during pregnancy and/or lactation and more particularly during pregnancy and lactation.

As used herein, unless otherwise indicated, a reference to administration during pregnancy (i.e. perinatal administration), particularly refers to administration of the composition comprising DGLA as defined herein , during any part of, or the whole of, the gestation period wherein a subject is pregnant with an offspring to which the prophylactic treatment is aimed e.g. The first week, the first two weeks, the first month, the first trimester, the second trimester or the third trimester of pregnancy. Particularly, the administration may be continued until at least the birth of the offspring. Thus, for example, in any embodiment of the present invention, administration during pregnancy refers to administration as soon as possible from conception (as defined above) until birth, i.e. during the full gestation period. In humans, the administration may be for a period of from: about 1 week to birth, about 2 weeks to birth, about 4 weeks to birth, about 8 weeks to birth, about 12 weeks to birth, about 18 weeks to birth.

As used herein, unless otherwise indicated, a reference to administration during lactation, particularly includes administration of the composition comprising DGLA postnatally at any time during which the offspring to which the prophylactic treatment is aimed is exclusively or partially ingesting the subject's maternal milk. For example administration during lactation may be for the period starting from onset of lactation until the end of the weaning process, i.e. when the offspring has ceased to ingest the maternal milk. During this period, the offspring may be exclusively or partially ingesting the maternal milk. More particularly, administration during lactation may refer to administration: for two weeks following the onset of lactation when the offspring is exclusively or partially ingesting the maternal milk. The administration during lactation may also include administration for a period of 1-24 months, 2-20 months, 3-18 months, 4-12 months or 4-8 months following the onset of lactation during which the offspring is exclusively or partially ingesting the maternal milk of the subject.

In any embodiment of the present invention the administration may be both prenatal for any period as defined above in relation to prenatal administration, as well as postnatal for any period as defined above in relation to the lactation period, and any combination of these periods as described above.

It may be particularly beneficial if administration is from 4 weeks of gestation or earlier to at least 6 months following the onset of lactation.

In any embodiment of the present invention the administration may be prenatal, i.e. at any period from conception to birth, as well as prenatal and/or postnatal during lactation, i.e. at any period from birth until the end of the weaning process, i.e. when the offspring has ceased to ingest the maternal milk, and any combination of these periods as described above.

In any aspect or any embodiment of the present invention, pre-pregnancy supplementation or administration preferably refers to administration from about 1-24 months, 1-18 months, 1-12 months, 1-6 months, or 1-3 months prior to pregnancy. Administration of the composition comprising DGLA pre-pregnancy may be particularly beneficial as it may enable the subject to build up an optimal amount of DGLA in the body from which the offspring of said subject may benefit in terms of prophylactic effect against allergic diseases.

Preferably, in any embodiment of the present invention, the offspring is not directly administered the DGLA. Thus, the composition comprising DGLA of the present invention, when administered prenatally to the subject is indirectly transmitted to the developing embryo or fetus, e.g., via the placenta or amniotic fluid. In other words, the exposure of the offspring to the DGLA is *in utero* when the DGLA is administered to the subject (mother) during pregnancy.

Similarly, the composition comprising DGLA of the invention, when administered postnatally to a lactating subject, is indirectly transmitted to the neonate or infant via the ingestion of maternal milk, i.e. the exposure of the offspring to the composition containing DGLA that is administered to the subject (mother), is solely via the subject's (mother's) milk.

The present invention will now be described in further details by the way of the following examples. The following examples serve to illustrate various features and embodiments of the present invention.

### EXAMPLES

### Example 1

### MATERIALS AND METHODS

Soybean oil -was obtained from Florin AG (Switzerland; lot number 280813). The oil's fatty acids consisted mainly of the saturated fatty acids palmitic acid (14%) and stearic acid (4%), of the n-6 PUFA LA (47%) and GLA (0.2%), oleic acid (27%), and the n-3 PUFA ALA (5%). DGLA, EPA and DHA were undetectable.

High DHA fish oil (containing 20-26% docosahexaenoic acid (DHA; 22:6 n3), and 7% eicosapentaenoic acid (EPA; 20:5 n3) was obtained from Sofinol SA (a subsidiary of Nestle corporation). Other major fatty acids consisted of palmitic acid (21%), palmitoleic acid (3%), stearic acid (5%), and oleic acid (19%). GLA and DGLA levels were below 0.2%, ARA content was ^{∼}1%.

DGLA oil, derived from the fungus Mortierella alpina, was obtained from Nippon Suisan Kaisha Ltd. The DGLA content was 35.6%. Other major fatty acids consisted of palmitic acid (16.3%), stearic acid (7.1%), oleic acid (8.2%), LA (6.4%), GLA (2.6%), behenic acid (2.5%) and lignoceric acid (8.6%). The n-3 PUFA content was very low, with only ALA being detectable (0.5%) and EPA and DHA being absent.

Pregnant mice were divided into 3 groups (3-7 pregnant mice per group) - (i) control, (ii) fish oil (reference), and (iii) fish oil + DGLA. The mice were fed a low-fat based diet (standard rodent diet), to which was added (per 95 gram of that diet):
(i) 5 g soybean oil (control group);
(ii) 5 g high DHA fish oil (FO group) (reference); or
(iii) 2.5 g fish oil + 2.5 g DGLA oil (DGLA group).

These diets were being fed to pregnant mice, from days 3-5 of pregnancy (a pregnancy lasts 21 days) until weaning of the pups (weaning period was 3 weeks).

From week two, the pups were able to nibble on food in the cages, so to avoid direct exposure of the pups to the diet, the mothers and their pups were fed the control diet, but the mothers were additionally fed each day via intragastric gavage: (i) 0.1 ml of the soy bean oil (reference), (ii) 0.1 ml of high DHA fish oil (reference), or (iii) 0.05 ml high DHA fish oil + 0.05 ml of DGLA oil. Hence, the pups never had direct access to the diets.

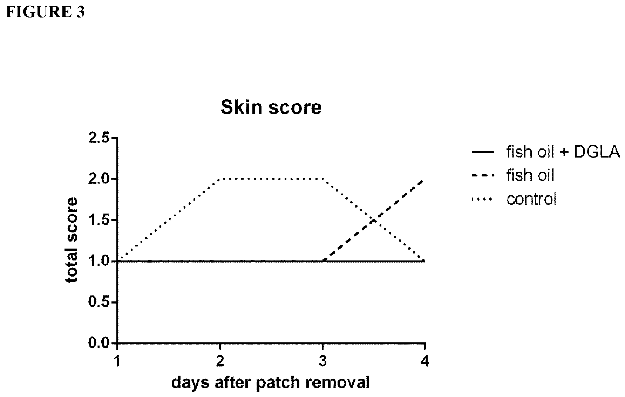

After weaning, all pups were put on the control diet. Two weeks after weaning, the pups (13 pups per group) received a skin patch with an allergen *Aspergillus fumigatus* extract, on a shaved part of their back, to induce allergy. The patch was removed after one week, and new patch was put on two weeks after removal of the first patch. The patch was removed and skin symptoms (signs of atopic dermatitis) were observed for the next 4 days (Figure 3). One day after the patch was removed, the mice also received an intranasal challenge with *Aspergillus* extract to see if an allergic response in the lung occurred.

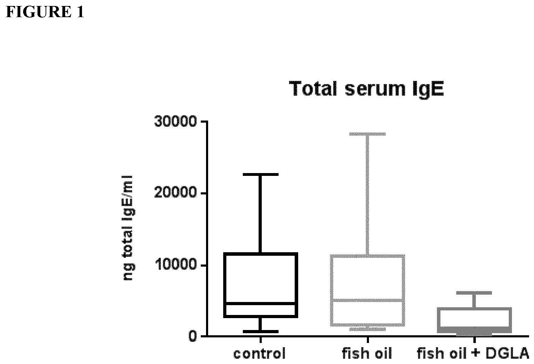

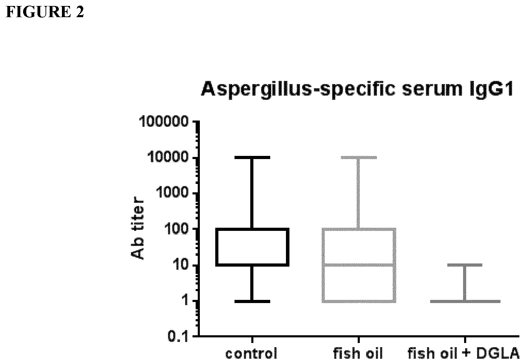

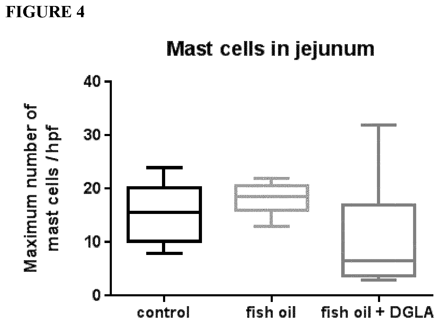

At the last day of skin assessment, the mice were humanely killed and tissue samples analyzed for antibodies in the serum - total IgE (Figure 1) and specific IgG1 (Figure 2), and for the presence of mast cells in the jejunum (Figure 4).

As shown in Figures 1 and 2, total IgE and specific IgG1 are significantly lower in the fish oil + DGLA group compared to the control and the fish oil only group (reference).

Moreover, as shown in Figure 3, skin symptoms were significantly milder in the fish oil + DGLA group than in the control, and moreover, the symptoms remained mild for a longer period in the fish oil + DGLA group compared with the fish oil only group (reference).

Further, the fish oil + DGLA group were found to have a significant lower number of mast cells in the jejunum compared with the fish oil only group and the control group.

### Example 2

### MATERIALS AND METHODS

RBL-2H3 cells; rat basophilic leukemia cells, were cultured under standard conditions with 15% serum in their culture medium. Once sufficient numbers of cells were present in the dishes, sodium salts of various fatty acids were added directly added to the medium, and the cells were incubated with these fatty acids for 24h. During the last 18h of this incubation period, the cells also received phorbol 12-myristate 13-acetate ("PMA"; final concentration 50 ng/mL) and ionomycin ("IM"; final concentration 0.125 µM) to stimulate IL4 secretion by the cells.

### RESULTS

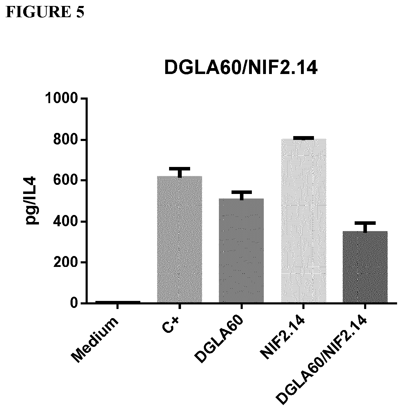

Figure 5 shows that cells incubated with DGLA (final concentration 60 µM) produce less IL4 upon stimulation with PMA and IM than cells not treated with additional fatty acids (control). The same was observed for cells incubated with 2.14 µM of a fatty acid mix ("NIF") consisting of DHA and EPA mixed in a 3:1 ratio. When DGLA and NIF were given together, a synergistic reduction of IL4 production was observed.

### Example 3

Pregnant mice were divided into 2 groups and fed, from day 5 of pregnancy until weaning of the pups, a low-fat based diet (standard rodent diet), to which was added (per 95 gram of that diet): 5 g soybean oil (control group) or 2.5 g fish oil + 2.5 g DGLA oil (fish oil + DGLA group). Oil compositions are described in Example 1.

From week two after birth, the pups were able to nibble on food in the cages, so to avoid direct exposure of the pups to the diet, the mothers and their pups were fed the control diet, but the mothers were additionally fed each day via intragastric gavage: 0.1 ml of the soy bean oil or 0.05 ml high DHA fish oil + 0.05 ml of DGLA oil. Hence, the pups never had direct access to the diets.

After weaning, all pups were put on the control diet. Two weeks after weaning, the pups (10 pups per group) received a skin patch as described in Example 1. At the last day of skin assessment, the mice were humanely killed and skin-draining, brachial lymph node collected. Brachial and mediastinal lymph nodes were homogenized using a syringe plunger in a cell strainer. The cells were centrifuged and washed two times with Roswell Park Memorial Institute (RPMI) medium (Sigma) supplemented with 10% fetal bovine serum, 1% L-glutamine, 1% penicillin/streptomycin and 0.1% gentamicin, and 50mM b-mercaptoethanol. The cells (3E5 cells/well) were cultured in a ninety-six-well flat-bottom plate in the absence or presence of Af. After 72 h of culture, the plates (including the supernatant and cells) were frozen until analysis of the supernatants.

The concentrations of IL-10 in cell-culture supernatants were determined with an electro-chemiluminescence-based multiplex assay (Mesoscale) according to the manufacturer's instructions.

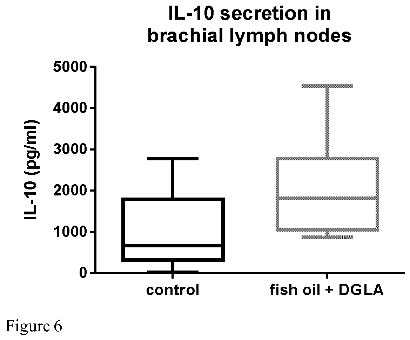

As shown in Figure 6, the immune-regulatory, anti-inflammatory cytokine IL-10 was significantly increased in pups from the fish oil + DGLA group as compared to pups from mothers fed a control diet. The increased levels of IL-10 might be might have contributed to the decreased skin score observed after patch removal (Figure 3). Alternatively, IL-10 could be an indicator of an anti-inflammatory mechanism induced in the pups by fish oil + DGLA during pregnancy and lactation to dampen the allergic response to Aspergillus later in life.

## Claims

1. A composition comprising DGLA for use in the prophylaxis of allergic disease in an offspring of a mammalian subject, comprising administration of the composition to said subject pre-pregnancy and/or during pregnancy and/or during lactation and preferably wherein said composition is a composition enriched in DGLA; wherein said composition also contains an omega-3 polyunsaturated fatty acid, selected from the group consisting of DHA and EPA or a combination of DHA and EPA; wherein said DGLA is comprised in said composition in a concentration of at least 3wt% relative to the total fatty acid content of the composition and more preferably in a concentration of at least 5wt%, at least 10wt%, at least 20wt%, at least 30wt%, at least 35wt%, or at least 40wt% relative to the total fatty acid content of the composition; and wherein the concentration of DGLA is greater than the concentration of DHA or EPA.

2. A composition comprising DGLA for use according to claim 1 wherein said composition further comprises omega-6 polyunsaturated fatty acid selected from the group consisting of LA and GLA or a combination of LA and GLA.

3. A composition comprising DGLA for use according to claim 2 wherein said composition comprises DGLA, GLA and LA and wherein the concentration of DGLA is greater than GLA and the concentration of GLA is greater than the concentration of LA.

4. A composition comprising DGLA for use according to anyone of claims 1 to 3 wherein said composition is a maternal nutritional composition and preferably selected from the group consisting of: pre-pregnancy supplement, pregnancy supplement or lactation supplement.

5. A composition comprising DGLA for use according to any of claims 1 to 4 wherein the allergic disease is selected from the group consisting of: an atopic disorder including hereditary atopic disorder and a Type 1 allergic disease including IgE mediated allergic disease, preferably wherein the allergic disease is selected from the group consisting of: asthma, allergic arthritis, allergic asthma, allergic bronchitis, allergic conjunctivitis, allergic keratitis, allergic rhinitis, allergic sinusitis, alimentary allergy, allergic respiratory disease, animal dander allergy, atopic dermatitis, atopic eczema, atopy, bronchial asthma, contact dermatitis, dermatitis, drug allergy, eczema, food allergy (particularly selected from the group consisting of egg allergy, fish allergy, milk allergy, nut allergy, shellfish allergy, soya allergy, and wheat allergy), food hypersensitivity, eosinophilic esophagitis, hayfever, house dust mite allergy, hypersensitivity pneumonitis, hypertrophic rhinitis, insect allergy, latex allergy, mould allergy, pruritus, seasonal allergic rhinitis, and vasomotor rhinitis.

## Patentansprüche

1. Zusammensetzung, umfassend DGLA, zur Verwendung bei der Prophylaxe einer allergischen Erkrankung bei einem Nachkommen eines Säugersubjekts, umfassend eine Verabreichung der Zusammensetzung an das Subjekt vor Schwangerschaft und/oder während Schwangerschaft und/oder während Stillzeit, und wobei die Zusammensetzung vorzugsweise eine Zusammensetzung ist, die mit DGLA angereichert ist; wobei die Zusammensetzung auch eine mehrfach ungesättigte Omega-3-Fettsäure enthält, ausgewählt aus der Gruppe, bestehend aus DHA und EPA oder einer Kombination von DHA und EPA; wobei die DGLA in der Zusammensetzung in einer Konzentration von mindestens 3 Gew.-% bezogen auf den Gesamtfettsäuregehalt der Zusammensetzung und mehr bevorzugt in einer Konzentration von mindestens 5 Gew.-%, mindestens 10 Gew.-%, mindestens 20 Gew.-%, mindestens 30 Gew.-%, mindestens 35 Gew.-% oder mindestens 40 Gew.-% bezogen auf den Gesamtfettsäuregehalt der Zusammensetzung enthalten ist; und wobei die Konzentration von DGLA größer als die Konzentration von DHA oder EPA ist.

2. Zusammensetzung, umfassend DGLA, zur Verwendung nach Anspruch 1, wobei die Zusammensetzung ferner mehrfach ungesättigte Omega-6-Fettsäure umfasst, ausgewählt aus der Gruppe, bestehend aus LA und GLA oder einer Kombination von LA und GLA.

3. Zusammensetzung, umfassend DGLA, zur Verwendung nach Anspruch 2, wobei die Zusammensetzung DGLA, GLA und LA umfasst und wobei die Konzentration von DGLA größer als GLA ist und die Konzentration von GLA größer als die Konzentration von LA ist.

4. Zusammensetzung, umfassend DGLA, zur Verwendung nach einem der Ansprüche 1 bis 3, wobei die Zusammensetzung eine maternale Nährstoffzusammensetzung ist und vorzugsweise aus der Gruppe ausgewählt ist, bestehend aus: Vorschwangerschaftsergänzung, Schwangerschaftsergänzung oder Stillzeitergänzung.

5. Zusammensetzung, umfassend DGLA, zur Verwendung nach einem der Ansprüche 1 bis 4, wobei die allergische Erkrankung aus der Gruppe ausgewählt ist, bestehend aus: einer atopischen Störung, einschließlich erblicher atopischer Störung, und einer allergischen Erkrankung vom Typ 1, einschließlich IgEvermittelter allergischer Erkrankung, wobei die allergische Erkrankung vorzugsweise aus der Gruppe ausgewählt ist, bestehend aus:
Asthma, allergischer Arthritis, allergischer Bronchitis, allergischer Konjunktivitis, allergischer Keratitis, allergischer Rhinitis, allergischer Sinusitis, Nahrungsmittelallergie, allergischer Atemwegserkrankung, Tierschuppenallergie, atopischer Dermatitis, atopischem Ekzem, Atopie, Bronchialasthma, Kontaktdermatitis, Dermatitis, Arzneimittelallergie, Ekzem, Lebensmittelallergie (insbesondere aus der Gruppe ausgewählt, bestehend aus Eiallergie, Fischallergie, Milchallergie, Nussallergie, Schalentierallergie, Sojaallergie und Weizenallergie), Nahrungsmittelüberempfindlichkeit, eosinophiler Ösophagitis, Heuschnupfen, Hausstaubmilbenallergie, Überempfindlichkeitspneumonitis, hypertropher Rhinitis, Insektenallergie, Latexallergie, Schimmelpilzallergie, Pruritus, saisonaler allergischer Rhinitis und vasomotorischer Rhinitis.

## Revendications

1. Composition comprenant du DGLA pour utilisation dans la prophylaxie d'une maladie allergique chez une progéniture d'un sujet mammalien, comprenant l'administration de la composition audit sujet avant la grossesse et/ou pendant la grossesse et/ou pendant l'allaitement et de préférence dans laquelle ladite composition est une composition enrichie en DGLA ; dans laquelle ladite composition contient également un acide gras polyinsaturé oméga-3, choisi dans le groupe constitué de DHA et d'EPA ou d'une combinaison de DHA et d'EPA ; dans laquelle ledit DGLA est compris dans ladite composition en une concentration d'au moins 3 % en poids par rapport à la teneur totale en acide gras de la composition et plus préférablement en une concentration d'au moins 5 % en poids, au moins 10 % en poids, au moins 20 % en poids, au moins 30 % en poids, au moins 35 % en poids, ou au moins 40 % en poids par rapport à la teneur totale en acide gras de la composition ; et dans laquelle la concentration en DGLA est supérieure à la concentration en DHA ou EPA.

2. Composition comprenant du DGLA pour utilisation selon la revendication 1 dans laquelle ladite composition comprend en outre un acide gras polyinsaturé oméga-6 choisi dans le groupe constitué de LA et de GLA ou d'une combinaison de LA et GLA.

3. Composition comprenant du DGLA pour utilisation selon la revendication 2 dans laquelle ladite composition comprend du DGLA, du GLA et du LA et dans laquelle la concentration en DGLA est supérieure à celle en GLA et la concentration en GLA est supérieure à la concentration en LA.

4. Composition comprenant du DGLA pour utilisation selon l'une quelconque des revendications 1 à 3 dans laquelle ladite composition est une composition nutritionnelle maternelle et est choisie de préférence dans le groupe constitué de : complément avant la grossesse, complément pour la grossesse ou complément pour l'allaitement.

5. Composition comprenant du DGLA pour utilisation selon l'une quelconque des revendications 1 à 4 dans laquelle la maladie allergique est choisie dans le groupe constitué de : un trouble atopique incluant un trouble atopique héréditaire et une maladie allergique de Type 1 incluant une maladie allergique à médiation IgE, de préférence dans laquelle la maladie allergique est choisie dans le groupe constitué de :
asthme, arthrite allergique, asthme allergique, bronchite allergique, conjonctivite allergique, kératite allergique, rhinite allergique, sinusite allergique, allergie alimentaire, maladie respiratoire allergique, allergie aux phanères d'animaux, dermatite atopique, eczéma atopique, atopie, asthme bronchique, dermatite de contact, dermatite, allergie aux médicaments, eczéma, allergie alimentaire (particulièrement choisie dans le groupe constitué d'une allergie aux œufs, d'une allergie au poisson, d'une allergie au lait, d'une allergie aux fruits à coque, d'une allergie aux crustacés, d'une allergie au soja et d'une allergie au blé), hypersensibilité alimentaire, œsophagite éosinophile, rhume des foins, allergie aux acariens, pneumonie d'hypersensibilité, rhinite hypertrophique, allergie aux insectes, allergie au latex, allergie aux moisissures, prurit, rhinite allergique saisonnière et rhinite vasomotrice.
